# EUROPEAN PATENT APPLICATION

(11) **EP 4 226 996 A1**
(43) Date of publication of application: **16.08.2023**
(21) Application number: 21886873.5
(22) Date of filing: 29.10.2021
(51) Int. Cl.: B01J 2/16, C12N 1/20, F26B 3/06, F26B 25/04, F26B 17/02

(54) **METHOD FOR PRODUCING AMINO ACID-MIXED SOLIDS AND APPARATUS FOR PRODUCING AMINO ACID-MIXED SOLIDS**

(30) Priority: 29.10.2020 KR 20200142144
(71) Applicant: CJ Cheiljedang Corporation, Seoul 04560 (KR)
(72) Inventor: JEONG, Daeyoung, Seoul 04560 (KR); LEE, In Sung, Seoul 04560 (KR); GWAK, Won Sik, Seoul 04560 (KR); YU, Jae Hun, Seoul 04560 (KR); KWON, Min Kyung, Seoul 04560 (KR); HONG, Jin Tae, Seoul 04560 (KR); KANG, Ji-hun, Seoul 04560 (KR)
(74) Representative: Regimbeau
(86) International application number: PCT/KR2021/015388
(87) International publication number: WO 2022/092879

(57) **Abstract**

The present disclosure relates to a method for preparing an amino acid mixed solid, including a first step of preparing an amino acid mixed solution containing an amino acid; a second step of stirring the amino acid mixed solution to form a thin film, and drying and pulverizing the formed thin film to prepare wet granules; and a third step of drying the wet granules to prepare an amino acid mixed solid in a granular formulation, wherein, the second step is conducted for drying the amino acid mixed solution so that a solid concentration in the wet granules is within a granulation concentration range.

## Description

### [Technical Field]

The present disclosure relates to a method for preparing an amino acid mixed solid and an apparatus for preparing an amino acid mixed solid.

### [Background Art]

L-Amino acids are a basic constituent unit of protein and are used as an important material for pharmaceutical raw materials, food additives, animal feeds, nutrients, insecticides, and bactericides. A target L-amino acid is produced mainly by a fermentation method using a microorganism developed by an artificial mutation method or a genetic recombination method or an artificial mutant strain thereof.

However, as a result of fermentation, not only the target L-amino acid but also by-products and waste are generated; therefore, in order to obtain a specific L-amino acid with high purity, it is essentially necessary to separate and purify the L-amino acid after the fermentation process. Meanwhile, since other products included in a fermentation product also contain nutritionally valuable components, demand for goods containing the entire fermentation product has increased, and in particular, there has been a demand for goods in the form of granules that are convenient to store, carry, and consume.

In order to granulate a fermentation product, granules are prepared after the water in the fermentation product is evaporated, and a large amount of energy is required to dry the fermentation product. This energy requirement leads to a decrease in efficiency of the granulation process. When a fermentation product is granulated using a fluidized-bed granulator or a mixed-type granulator according to the prior art (US Patent Application Publication No. 2015-0283527 and the like), there is a limit to increasing the solid content by concentrating the fermentation product, and this leads to a decrease in process efficiency.

With this technical background, the present inventors have established a simpler and more economical granule preparation process capable of increasing the amino acid mixed solid production efficiency by utilizing a thin film drying apparatus in the granulation process, and thereby completed the present disclosure based thereon.

### [Disclosure]

### [Technical Problem]

An object of the present disclosure is to provide a method and an apparatus by which an amino acid mixed solid in a granular formulation can be prepared at high efficiency by concentrating an amino acid mixed solution so that the solid concentration in the amino acid mixed solution is within a granulation concentration range and using the concentrated amino acid mixed solution.

### [Technical Solution]

The technical solution specifically described as follows. Meanwhile, each description and embodiment disclosed in this disclosure may also be applied to other descriptions and embodiments. That is, all combinations of various elements disclosed in the present disclosure fall within the scope of the present disclosure. Further, the scope of the present disclosure is not limited by the specific description below.

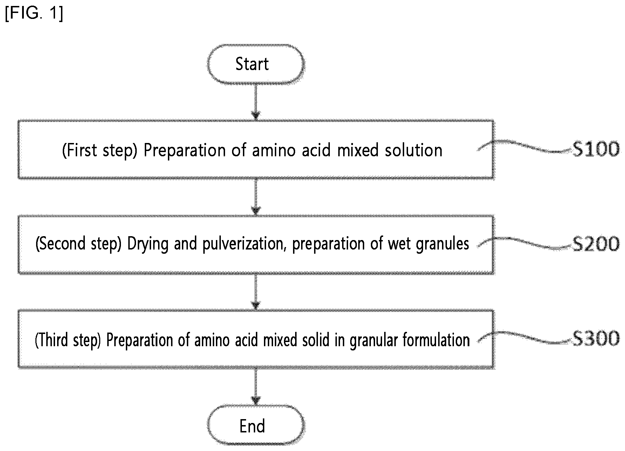

FIG. 1 is a flow chart illustrating a method for preparing an amino acid mixed solid according to an aspect of the present disclosure.

According to an aspect of the present disclosure, there is provided a method for preparing an amino acid mixed solid, including a first step (S100) of preparing an amino acid mixed solution containing an amino acid; a second step (S200) of stirring the amino acid mixed solution to form a thin film, and drying and pulverizing the formed thin film to prepare wet granules; and a third step (S300) of drying the wet granules to prepare an amino acid mixed solid in a granular formulation, wherein the second step is conducted for drying the amino acid mixed solution so that a solid concentration in the wet granules is within a granulation concentration range in the second step (S200).

By utilizing the method for preparing an amino acid mixed solid described above, it is possible to dry an amino acid mixed solution so that the solid concentration in the amino acid mixed solution is within the granulation concentration range and then prepare a granular formulation.

Hereinafter, the respective steps in the method for preparing an amino acid mixed solid of the present disclosure will be described.

First, a first step (S100) of preparing an amino acid mixed solution containing an amino acid is performed.

In the first step (S100), the amino acid may be an L-amino acid, and may be prepared by an extraction method, a synthesis method, a fermentation method, an enzyme method, and the like. In some cases, the amino acid may be supplied from a fermentation product prepared by a fermentation method using a microorganism. At this time, the term "fermentation product" in the present disclosure may refer to a resulting product of enzymatic or metabolic synthesis or decomposition of organic substances using microorganisms. For example, the fermentation product may include a culture itself containing microorganisms, which is obtained by culturing microorganisms in a culture medium, or a concentrate, dried product, or freeze-dried product of a culture obtained by removing microorganisms therefrom. In this case, the amino acid mixed solution may be one which contains an L-amino acid and the entire fermentation product, or one which is obtained by removing impurities from the fermentation product.

The fermentation product prepared to supply an amino acid in the first step (S100) may be a resulting product obtained by culturing a microorganism that produces an L-amino acid. At this time, the term "microorganism that produces an L-amino acid" in the present disclosure includes both wild-type microorganisms and microorganisms in which natural or artificial genetic modification has occurred, and may be a microorganism in which a specific mechanism is weakened or enhanced by causes such as insertion of an external gene or enhancement or inactivation of the activity of an endogenous gene, containing genetic modification for the production of a target protein or product.

The above-described microorganism used in the first step (S100) may be at least one selected from microorganisms of the genus *Brevibacterium, Corynebacterium*, *Escherichia*, *Serratia*, *Erwinia*, *Enterobacteria*, *Streptomyces* or *Pseudomonas* or artificial mutants thereof. Hence, the first step (S100) may further include a step of preparing an amino acid mixed solution using at least one of the above-described microorganisms.

Among the above-described microorganisms of the present disclosure that can be used in the first step (S100), the "microorganisms of the genus *Corynebacterium*" may include all microorganisms of the genus *Corynebacterium*. Specifically, the microorganism may be *Corynebacterium glutamicum*, *Corynebacterium crudilactis, Corynebacterium deserti, Corynebacterium efficiens*, *Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris* or *Corynebacterium flavescens*, and more specifically, the microorganism may be *Corynebacterium glutamicum*, but is not limited thereto.

Microorganisms of the genus *Corynebacterium*, particularly *Corynebacterium glutamicum*, are Gram-positive microorganisms that are widely used in the production of L-amino acids and other useful substances. In order to produce the L-amino acids and other useful substances, various studies are being conducted to develop microorganisms for highly efficient production and fermentation process technology. For example, target substance-specific approaches such as increasing the expression of genes encoding enzymes involved in biosynthesis of L-tryptophan, L-valine, L-threonine, L-isoleucine, L-leucine and the like, or removing genes unnecessary for biosynthesis are mainly used.

In the first step (S100), a step of preparing a fermentation product containing an L-amino acid using the above-described microorganism, and then separating and purifying the fermentation product to recover an amino acid mixed solution may be further performed. The recovery of amino acid mixed solution may be to collect an amino acid mixed solution containing the target L-amino acid using a suitable method known in the art according to the microorganism culturing method of the present disclosure, for example, a batch, continuous or fed-batch culture method. For this purpose, centrifugation, filtration, treatment with a crystallized protein precipitating agent (salting out method), extraction, ultrasonic disruption, ultrafiltration, dialysis, various kinds of chromatography such as molecular sieve chromatography (gel filtration), adsorption chromatography, ion exchange chromatography, and affinity chromatography, HPLC, and a combination of these methods may be used, and the target amino acid mixed solution may be recovered from the medium or microorganism using suitable methods known in the art.

Recovering an amino acid mixed solution from a fermentation product in the first step (S100) does not necessarily mean that only the L-amino acid contained in the fermentation broth is separated and recovered. If necessary, the recovery may mean recovering the entire fermentation product or recovering an amino acid mixed solution, which is obtained by removing only some impurities from the fermentation product and thus contains an L-amino acid, the microorganism used in the culture, or the like.

The amino acid mixed solution supplied in the first step (S100) may further contain any suitable excipient commonly used in compositions for amino acid production, and such an excipient may be, for example, preservatives, wetting agents, dispersing agents, suspending agents, buffers, stabilizers or isotonic agents, but is not limited thereto.

After the first step (S100) has been performed and before the second step (S200) is performed, a process of concentrating the amino acid mixed solution may be further performed. The concentration apparatus used in the concentration process is not limited. For example, a forced circulation concentration pipe may be used, and a paddle dryer, a slurry drying facility and the like may be used in the concentration process, but the concentration apparatus is not limited thereto.

Next, in the second step (S200), a thin film is formed by stirring the amino acid mixed solution using a thin film drying apparatus, and the formed thin film is dried and pulverized to prepare wet granules. The thin film drying apparatus is an apparatus capable of simultaneously stirring and drying fluid, and may be operated in a form of stirring the fluid introduced into the chamber to form a thin film composed of the fluid on the chamber wall and drying the formed thin film. Additionally, the dried thin film may be pulverized into granules again and discharged out of the thin film drying apparatus. The thin film drying apparatus may be of a horizontal type in which a chamber is equipped parallel to the ground or a vertical type in which a chamber is equipped perpendicular to the ground, but is not limited thereto.

In the second step (S200), at least part of the water contained in the amino acid mixed solution may be evaporated and removed. Accordingly, while the amino acid mixed solution is dried, the solid concentration in the amino acid mixed solution may increase, and thereby wet granules may be provided.

In the wet granules prepared in the second step (S200), "granule" is a macroscopic particle that is a permanent aggregate of a larger size formed by gathering small particles such as powder, and the small particles constituting the aggregate can still be distinguished from one another in the aggregate. The powder or the like aggregates to form particles that are about 30 to 150 times larger than the powder, and the particles may be redried and granulated. The grains formed by the granulation (i.e., the granules) have a porous structure, and thus have wettability and, at the same time, increased dispersion and sedimentation rates in water. In the present disclosure, the granulation may be granulation of the fermentation product being in a granular form in the process of drying the fermentation product, but is not limited thereto. Wet granules may refer to granules that are in the above-described granular form but in a state containing water.

In the second step (S200), moisture drying is performed so that the solid concentration in the amino acid mixed solution is within the granulation concentration range. At this time, the granulation concentration range means a solid concentration range that allows the amino acid mixed solid in a granular formulation to be prepared in the third step (S300).

The granulation concentration range described in the second step (S200) may vary depending on the kind of amino acids. Specifically, the amino acid may include at least one selected from the group consisting of L-threonine, L-tryptophan, L-valine, L-isoleucine, and L-leucine, and the granulation concentration range may vary depending on the kind and composition of amino acid contained in the amino acid mixed solution. Specifically, in a case where the amino acid is L-tryptophan, the granulation concentration range may be about 80% to about 92% by weight. In a case where the amino acid is L-valine, the granulation concentration range may be about 80% to about 94% by weight. In a case where the amino acid is L-threonine, the granulation concentration range may be about 85% to about 96% by weight. In a case where the amino acid is L-isoleucine, the granulation concentration range may be about 82% to about 94% by weight. In a case where the amino acid is L-leucine, the granulation concentration range may be about 82% to about 93% by weight. In a case where plural kinds of amino acids are mixed in the amino acid solution, the granulation concentration range may be adjusted in consideration of the kinds of amino acids mixed and the contents of the respective amino acids.

Hence, in performing the second step (S200), the granulation concentration range may be controlled based on the information on the kind and composition of amino acids contained in the amino acid mixed solution supplied from the first step (S100). In the second step (S200), the drying process may be performed depending on the determined granulation concentration range.

The amount of steam used in the third step (S300) may be reduced by supplying the wet granules from which water has been removed in the second step (S200). In order to granulate the fermentation product, granulation can be performed after all water in the fermentation product has been evaporated. According to the prior art, part of water contained in the fermentation product is removed through a concentration process before the granulation process is performed. Specifically, in the existing granule preparing methods, it is required that the content of solids contained in the slurry be adjusted to about 40% to 55% in the case of amino acids exhibiting high solubility and the content of solids be adjusted to about 18% to 22% in the case of amino acids exhibiting low solubility. According to the present disclosure, by drying the amino acid mixed solution to the granulation concentration range in the second step (S200), the amount of steam used in the subsequent third step (S300) can be reduced and productivity and production efficiency in the entire preparation process can be significantly improved.

The linear speed to stir the amino acid mixed solution in the second step (S200) may be about 4 m/s to about 17 m/s, about 4 m/s to about 16 m/s, about 4 m/s to about 15 m/s, about 4 m/s to about 14 m/s, about 5 m/s to about 17 m/s, about 5 m/s to about 16 m/s, about 5 m/s to about 15 m/s, or about 5 m/s to about 14 m/s. In a case where the linear speed to stir the amino acid mixed solution is less than the above-mentioned range, the formation rate of a thin film provided from the amino acid mixed solution in the thin film drying apparatus in which the second step (S200) is performed is improved, and thus drying-stirring can be performed without a stirrer load during drying. By drying the amino acid mixed solution to an appropriate level without an over-drying problem, it is possible to provide a particle size so that the particles are not lost to the bag filter and thus to improve the granule recovery rate.

The second step (S200) may be performed at a pressure of about 0.05 atm to about 0.6 atm, about 0.05 atm to about 0.5 atm, or about 0.05 atm to about 0.4 atm. Since the second step (S200) is performed under a relatively low pressure as described above, the vapor pressure of water contained in the amino acid mixed solution is lowered, and the water can be evaporated at a temperature lower than 100°C. Hence, it is not required to heat the amino acid mixed solution to a high temperature to remove water in the second step (S200), and there is thus no concern that substances contained in the amino acid mixed solution, such as L-amino acids and microorganisms, are denatured during the heating process for moisture drying. Meanwhile, in a case where the pressure for performing the second step (S200) is less than the value in the above-described range, there is a concern that dust may pass to the bag filter of the thin film drying apparatus and the yield may decrease. In a case where the pressure for performing the second step (S200) exceeds the value in the above-described range, a high temperature is required for moisture drying, and browning of the granules due to the high temperature may occur. Since a relatively small amount of heat is required during the drying process, a small amount of energy is consumed. Hence, by maintaining the pressure for performing the second step (S200) within the above-described range, granules can be prepared in high yield without denaturation of L-amino acids or microorganisms.

The distance between the inner wall and the stirring unit may be about 5 m to about 15 mm. Formation of granules can be efficiently performed when the distance is within the above range.

As the particle size of the wet granular particles obtained in the second step (S200), particles having a size of 2,000 µm or more may be 5.0% or less, specifically 3.0% or less, more specifically 1.0% or less, and particles having a size of 75 µm or less may be 20.0% or less, specifically 15.0% or less, more specifically 5.0% or less, but the particle size is not limited thereto.

Next, a third step (S300) of drying the wet granules prepared in the second step (S200) to prepare an amino acid mixed solid in a granular formulation may be performed. The third step (S300) may be performed using a fluidized-bed granulator/dryer. The amino acid mixed solid in a granular formulation may be prepared by supplying wet granules into a fluidized-bed granulator/dryer and drying the wet granules through the flow of air. Meanwhile, the third step (S300) does not necessarily have to be performed only by a fluidized-bed granulator/dryer.

The third step (S300) may further include a step of sieving the amino acid mixed solid in a granular formulation formed by drying. At this time, the amino acid mixed solid in a granular formulation may be sieved based on the desired particle size. The particle size serving as the sieving standard may be appropriately selected according to the choice of those skilled in the art. For example, the particle size as the sieving standard is about 50 µm to about 3,000 µm, more specifically about 75 µm to about 2,000 µm, still more specifically about 100 µm to about 2,000 µm, but is not limited thereto.

After the third step (S300) has been performed, a process of pulverizing and/or circulating granular particles not having the desired particle size may be further performed in order to reuse the particles remained after being sieved as described above. For example, the particles remained after being sieved may include particles having a particle size of 2000 µm or more at 1.0% or less of the total particles and particles having a particle size of 75 µm or less at 1.5% or less of the total particles, but are not necessarily limited thereto.

Additionally, the method for pulverizing and/or circulating the granular particles is not particularly limited, and methods known in the art may be used.

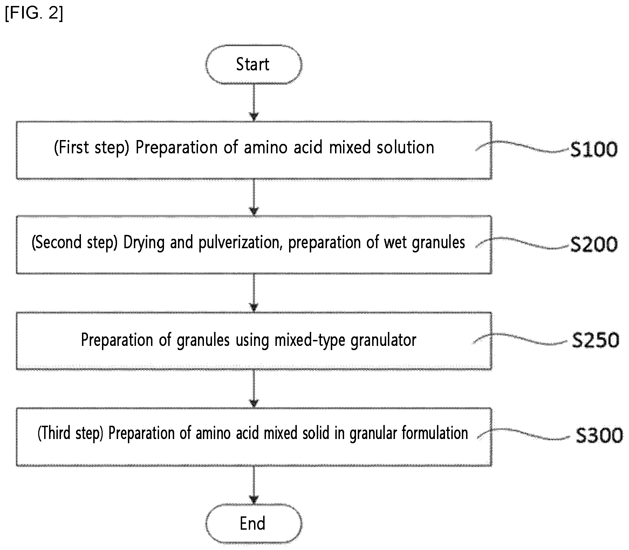

FIG. 2 is a flow chart illustrating the method for preparing an amino acid mixed solid according to the present disclosure.

Referring to FIG. 2, between the second step (S200) and the third step (S300), a step (S250) of preparing granules using a mixed-type granulator may be further performed.

In the step of using a mixed-type granulator (S250), at least a portion of the wet granules prepared in the second step (S200) may be used as a seed for preparing granules in the mixed-type granulator. In the mixed-type granulator, the amino acid mixed solution prepared in the first step (S100) may be sprayed onto the wet granules prepared through pulverization in the second step (S200), and thus granules of an appropriate size may be prepared. The granules prepared in the mixed-type granulator may be dried in the third step (S300), and thus an amino acid mixed solid in a granular formulation may be provided.

In the above, the method for preparing an amino acid mixed solid according to the present disclosure has been described. Hereinafter, the preparation apparatus of the present disclosure that can be used in the above-described method for preparing an amino acid mixed solid will be further described.

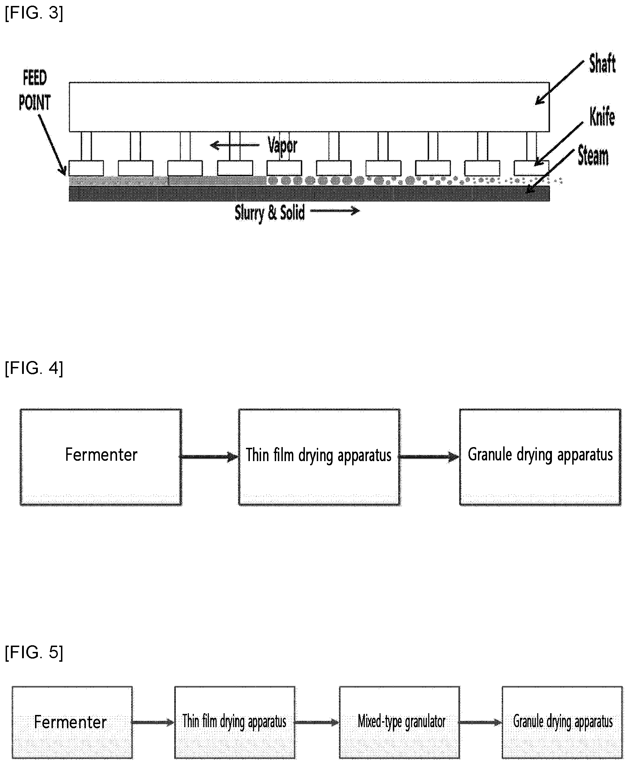

FIG. 3 is a diagram illustrating a thin film drying apparatus according to the present disclosure.

Referring to FIG. 3, the apparatus for preparing an amino acid mixed solid includes a fermenter for producing an amino acid mixed solution through a fermentation process, a thin film drying apparatus for drying and pulverizing the amino acid mixed solution supplied from the fermenter to prepare wet granules, and a granule drying apparatus for drying the wet granules to prepare an amino acid mixed solid in a granular formulation.

First, the fermenter is a member for supplying a fermentation product containing an amino acid by a fermentation method using a microorganism. In the description of the fermenter, the microorganism, fermentation method, amino acid, and fermentation product are as described above.

The fermenter may include a container for performing fermentation, an introduction unit for introducing raw materials, a discharge unit for discharging the fermentation product upon completion of fermentation to the outside, a stirring unit for stirring raw materials in the container, and a heating unit for applying heat to cause a fermentation reaction.

The fermenter may further include a member for collecting an amino acid mixed solution containing an L-amino acid from the fermentation product inside or outside the container. For example, members for performing centrifugation, filtration, treatment with a crystallized protein precipitating agent (salting out method), extraction, ultrasonic disruption, ultrafiltration, dialysis, various kinds of chromatography such as molecular sieve chromatography (gel filtration), adsorption chromatography, ion exchange chromatography, and affinity chromatography, and HPLC may be further equipped inside or outside the fermenter.

The amino acid mixed solution collected from the fermentation product produced in the fermenter is transferred to the thin film drying apparatus. At this time, before fermentation product is transferred from the fermenter to the thin film drying apparatus, the amino acid mixed solution may be concentrated in a concentration member. The concentration apparatus is not limited. For example, a forced circulation concentration pipe may be used, and a paddle dryer, a slurry drying facility, or the like may be used in the concentration process, but the concentration apparatus is not limited thereto.

Wet granules may be prepared by drying and pulverizing the amino acid mixed solution supplied from the fermenter in the thin film drying apparatus. The thin film drying apparatus sprays and simultaneously stirs the amino acid mixed solution in a liquid or slurry state inside the apparatus so that a thin film provided from the amino acid mixed solution can be formed on the inner wall of the thin film drying apparatus. The thin film drying apparatus also heats the inner wall so that the thin film formed on the inner wall can be dried. The dried thin film is pulverized and thus wet granules are prepared.

The thin film drying apparatus may be equipped in a horizontal type parallel to the ground or a vertical type perpendicular to the ground. In the drawing, a thin film drying apparatus of a horizontal type parallel to the ground is equipped, but a thin film drying apparatus of a vertical type may be used if necessary. In addition, the aspect ratio of the thin film drying apparatus may also vary depending on the form of thin film drying apparatus equipped. For example, in a case where the thin film drying apparatus is equipped in a horizontal type parallel to the ground, the length in the transverse direction (direction parallel to the ground) may be longer than the length in the longitudinal direction (direction perpendicular to the ground). In contrast, in a case where the thin film drying apparatus is equipped in a vertical type perpendicular to the ground, the length in the transverse direction may be shorter than the length in the longitudinal direction. The form of the thin film drying apparatus may be configured differently depending on the quipped form and use of the thin film drying apparatus.

The thin film drying apparatus includes a stirring unit and a heating unit.

The stirring unit of the thin film drying apparatus stirs the amino acid mixed solution introduced into the thin film drying apparatus. The amino acid mixed solution stirred by the thin film drying apparatus stirring unit is applied onto the inner wall of the thin film drying apparatus by centrifugal force, and is provided in the form of a thin film on the inner wall.

The thin film formed through stirring by the stirring unit of the thin film drying apparatus is dried on the inner wall. Specifically, the thin film may be dried on the inner wall by the heating unit that transmits heat to the inner wall.

The stirring unit of the thin film drying apparatus may further include a plurality of blades. The blades in the stirring unit of the thin film drying apparatus can push the amino acid mixed solution introduced into the thin film drying apparatus toward the inner wall to form a thin film while capable of separating the dried thin film from the inner wall or pulverize the dried thin film. Specifically, the blades in the stirring unit of the thin film drying apparatus located adjacent to the inlet of the thin film drying apparatus (the region where the amino acid mixed solution is supplied) may be used to push the amino acid mixed solution toward the inner wall to form a thin film, and the blades in the stirring unit of the thin film drying apparatus located adjacent to the outlet of the thin film drying apparatus (region where wet granules are discharged) may be used to separate the dried thin film from the inner wall and pulverize the dried thin film to form wet granules.

The stirring unit of the thin film drying apparatus (thin blades in the stirring unit of the thin film drying apparatus) may be equipped to be separated at a distance of 5 mm to 15 mm from the inner wall of the thin film drying apparatus. By isolating the stirring unit blades and the inner wall of the thin film drying apparatus by the above-mentioned range, it is possible to improve the efficiency of heat transmission to the amino acid mixed solution and decrease the motor load due to stirring.

The form of the heating unit is not limited. The heating unit may be a heat transmission member in direct contact with the inner wall, and is equipped in the form of a pipe in direct contact with the inner wall, and may transmit heat to the inner wall by supplying steam to the pipe. The heating unit may be equipped on the entire inner wall of the thin film drying apparatus. It is thus possible to simultaneously perform drying of the amino acid mixed solution or thin film in a large area, and to improve the drying efficiency.

Next, the wet granules prepared in the thin film drying apparatus are supplied into the granule drying apparatus. In the granule drying apparatus, the wet granules are dried to a desired humidity to prepare amino acid mixed solids in a granular formulation. The granule drying apparatus may, for example, supply dry hot air to dry the wet granules. However, the equipped form and operation form of the granule drying apparatus are not limited to the above examples.

FIGS. 4 and 5 are block diagrams illustrating a simplified apparatus for preparing an amino acid mixed solid according to an embodiment of the present disclosure.

Referring to FIG. 4, the apparatus for preparing an amino acid mixed solid according to the present disclosure may include a fermenter, a thin film drying apparatus, and a granule drying apparatus as described above. Specifically, the apparatus for preparing an amino acid mixed solid according to an embodiment of the present disclosure may include a fermenter for producing an amino acid mixed solution through a fermentation process; a thin film drying apparatus for drying and pulverizing the amino acid mixed solution supplied from the fermenter to prepare wet granules; and a granule drying apparatus for drying the wet granules to prepare an amino acid mixed solid in a granular formulation, and the thin film drying apparatus may include a stirring unit of the thin film drying apparatus for stirring the amino acid mixed solution to form a thin film provided from the amino acid mixed solution on the inner wall of the thin film drying apparatus; and a heating unit for heating the inner wall of the thin film drying apparatus to dry the thin film.

Referring to FIG. 5, the apparatus for preparing an amino acid mixed solid further includes a mixed-type granulator. When a mixed-type granulator is used, granules may be prepared by using at least a portion of the wet granules prepared in the thin film drying apparatus as a seed.

### [Advantageous Effects]

According to the present disclosure, granules can be formed after an amino acid mixed solution has been dried to a granulation concentration range, and it is thus possible to increase production volume and improve process efficiency by supplementing the formulation problem due to water in the existing purification methods. Hence, the present disclosure can greatly contribute to cost reduction in the production of amino acid granules.

### [Brief Description of Drawings]

FIG. 1 is a flow chart illustrating a method for preparing an amino acid mixed solid according to the present disclosure;
FIG. 2 is a flow chart illustrating a method for preparing an amino acid mixed solid according to the present disclosure;
FIG. 3 is a simplified cross-sectional view of a thin film drying apparatus according to the present disclosure; and
FIGS. 4 and 5 are block diagrams illustrating simplified method and apparatus for preparing an amino acid mixed solid according to an embodiment of the present disclosure.

### [Detailed Description of the Invention]

Hereinafter, the present disclosure will be described in more detail with reference to Examples. However, the following Examples are only preferred embodiments for illustrating the present disclosure and, therefore, are not intended to limit the scope of the present disclosure thereto. Meanwhile, technical matters not described in the present specification can be sufficiently understood and easily implemented by those skilled in the art of the present disclosure or similar technical fields.

### Example 1A. Preparation of amino acid mixed solid containing L-tryptophan

In order to prepare an amino acid mixed solid containing L-tryptophan by the method according to the present disclosure, an L-tryptophan fermentation broth was first recovered from the fermenter.

The recovered L-tryptophan fermentation broth had a composition of an L-tryptophan concentration of 57 g/L, purity of 67%, and solid content of 8.4%. The recovered L-tryptophan fermentation broth was introduced into a concentration pipe for pilot use (forced circulation type, manufacturer MAHN MIN Machinery Co., Ltd., Korea) and concentrated. The fermentation broth used for concentration was 750 L in total, and concentrated 5 times in accordance with the capacity of the concentration pipe, 150 L/1 time. Concentration was performed under concentration conditions of a pressure of 0.1 atm and a steam pressure of 3 atm until the solid concentration reached 25.5%. Concentration was performed only until the solid concentration reached 25.5% since gelation might occur and fluidity might be lost when the solid concentration was 25.5% or more. The concentration of the recovered concentrate was 178.9 g/L, the purity was 67%, the solid concentration was 25.5%, and the volume was 239 L. Into a thin film drying apparatus, 19.1 L of the recovered concentrate was supplied.

Wet granules were prepared by performing drying and formulation processes using a thin film drying apparatus (double jacket, horizontal type, manufacturer JANGWOO MACHINERY CO., LTD., Korea).. In Example 1A, the operating conditions of the thin film drying apparatus were set to an internal pressure of 0.2 atm, a steam pressure of 3 atm, a knife linear speed of 8 m/s, and a distance of 5 mm between the blades of the stirring unit of the thin film drying apparatus and the inner wall.

From the thin film drying apparatus, 5.48 kg of wet granules were recovered, and the recovered wet granules had a purity of 67%, solid content of 92%, and an average particle size of about 400 to 700 µm. The wet granules were placed in a fluidized-bed granulator/dryer (manufacturer GR Engineering) and dried. The recovery rate of the amino acid mixed solid recovered from the fluidized-bed granulator/dryer was 97.7%, the recovered amino acid mixed solid weighed 5.04 kg, the purity was 67%, and the solid content was 99%.

### Experimental Example 1-1. Comparison of results of preparation of amino acid mixed solid depending on solid content in wet granules recovered from thin film drying apparatus

In order to compare the results of preparation of amino acid mixed solid depending on the solid content in wet granules recovered from the thin film drying apparatus, amino acid mixed solids were prepared while varying the degree of moisture drying.

In Examples and Comparative Examples used in Experimental Example 1-1, 19.1 L of the same material as the concentrated L-tryptophan fermentation broth used in Example 1A was supplied into a thin film drying apparatus, respectively. The operating conditions of the thin film drying apparatus were maintained the same as in Example 1A, and the introduction speed was adjusted. Next, the recovered wet granules were placed in a fluidized-bed granulator/dryer and dried according to Examples and Comparative Examples.

Experimental results for each condition of Examples and Comparative Examples are as shown in Table 1 below.

**[Table 1]**

| Division | | Example 1A | Example 1B | Example 1C | Example 1D | Comparative Example 1A | Comparative Example 1B |
|---|---|---|---|---|---|---|---|
| Wet granules | Formatio n of granules | ○ | ○ | ○ | ○ | X | X |
| | Solid content | 92% | 88% | 85% | 80% | 75% | 96% |
| | Size | 400~700 µm | 400~700 µm | 500~800 µm | 500~800 µm | - | 200~300 µm |
| | Weight | 5.48 kg | 5.74 kg | 5.92 kg | 6.30 kg | - | 5.18 kg |
| Wet granules are placed in a laboratory fluidized-bed granulator/dryer and dried (discharged moisture 1%) | | | | | | | |
| Amino acid mixed solid | Weight | 5.04 kg | 5.05 kg | 5.02 kg | 5.03 kg | | 4.88 kg |
| | Yield | 97.7% | 98.0% | 97.4% | 97.5% | | 94.6% |

As a result of the experiment, it was confirmed that granules were not formed when the solid content in wet granules recovered from the thin film drying apparatus was less than 80%, for example, 75% or less. As the solid content in wet granules increased, the formation of granules became more difficult, and an additional formulation process was required. It was confirmed that granules were not formed when the solid content in wet granules was more than 92%, for example, 96% or more.

### Experimental Example 1-2. Comparison of results of preparation of amino acid mixed solid depending on stirring linear speed of thin film drying apparatus

In Examples and Comparative Examples used in Experimental Example 1-2, 19.1 L of the same material as the concentrated L-tryptophan fermentation broth used in Example 1A was supplied into a thin film drying apparatus (JANGWOO MACHINERY CO., LTD., horizontal type TFD), respectively. The stirring speed (linear speed) in the thin film drying apparatus was adjusted, and the operating conditions of the thin film drying apparatus except for the stirring speed were maintained the same as in Example 1A. Next, the recovered wet granules were placed in a fluidized-bed granulator/dryer and dried according to Examples and Comparative Examples.

Experimental results for each condition of Examples and Comparative Examples are as shown in Table 2 below.

**[Table 2]**

| Division | | Example 1A | Example 1E | Example 1F | Example 1G | Comparative Example 1C | Comparative Example 1D |
|---|---|---|---|---|---|---|---|
| Linear speed | | 8 m/s | 5 m/s | 11 m/s | 14 m/s | 18 m/s | 3 m/s |
| Wet granules | Formatio n of granules | ○ | ○ | ○ | ○ | X | X |
| | Solid content | 92% | 89% | 94% | 95% | 96% | - |
| | Size | 400~700 µm | 400~700 µm | 300~600 µm | 300~500 µm | 200~300 µm | - |
| | Weight | 5.48 kg | 5.67 kg | 5.33 kg | 5.25 kg | 5.14 kg | - |
| Wet granules are placed in a laboratory fluidized-bed granulator/dryer and dried (discharged moisture 1%) | | | | | | | |
| Amino acid mixed solid | Weight | 5.04 kg | 5.05 kg | 4.92 kg | 4.89 kg | 4.78 kg | - |
| | Yield | 97.7% | 97.9% | 95.5% | 94.9% | 92.7% | - |

As a result of the experiment, it was confirmed that as the stirring speed (linear speed) in the thin film drying apparatus increased, the amount of heat that the solids in the amino acid mixed solution can receive increased and the drying rate increased. However, when the linear speed exceeded 17 m/s, the amino acid mixed solution was overdried, the average particle size of wet granules decreased, and there was thus a problem in that the recovery rate decreased due to a significant loss of fine powder to the thin film drying apparatus and the bag filter of the fluidized-bed granulator/dryer. In contrast, at a linear speed of less than 4 m/s, the thin film formation rate decreased inside the thin film drying apparatus, and the load on the stirring unit of the thin film drying apparatus increased during drying and thus stirring was not performed.

### Experimental Example 1-3. Comparison of results of preparation of amino acid mixed solid depending on distance between stirring unit of thin film drying apparatus and inner wall in thin film drying apparatus

In Examples and Comparative Examples used in Experimental Example 1-3, 19.1 L of the same material as the concentrated L-tryptophan fermentation broth used in Example 1A was supplied into a thin film drying apparatus, respectively. The distance between the blades of the stirring unit of the thin film drying apparatus and the inner wall in the thin film drying apparatus was adjusted, and other operating conditions of the thin film drying apparatus were maintained the same as in Example 1A. Next, the recovered wet granules were placed in a fluidized-bed granulator/dryer and dried according to Examples and Comparative Examples.

Experimental results for each condition of Examples and Comparative Examples are as shown in Table 3 below.

**[Table 3]**

| Division | | Example 1A | Example 1H | Comparative Example 1E |
|---|---|---|---|---|
| Distance between inner wall and thin film drying apparatus stirring unit blade | | 5 mm | 15 mm | 25 mm |
| Wet granules | Formation of granules | ○ | ○ | X |
| | Solid content | 92% | 87% | - |
| | Size | 400~700 µm | 500~900 µm | - |
| | Weight | 5.48 kg | 5.81 kg | - |
| Wet granules are placed in a laboratory fluidized-bed granulator/dryer and dried (discharged moisture 1%) | | | | |
| Amino acid mixed solid | Weight | 5.04 kg | 5.04 kg | - |
| | Yield | 97.7% | 97.8% | - |

As a result of the experiment, it was confirmed that as the distance between the blades of the stirring unit of the thin film drying apparatus and the inner wall in the thin film drying apparatus became wider, the amount of the material to be dried increased, which slowed the heat transmission, and thus the drying rate decreased. As can be seen from Comparative Example 1E, when the distance exceeded about 15 mm, the load on the motor of the stirring unit of the thin film drying apparatus was increased by the dried product, thus making the operation impossible.

### Experimental Example 1-4. Comparison of results of preparation of amino acid mixed solid depending on pressure condition in thin film drying apparatus

In Examples and Comparative Examples used in Experimental Example 1-4, 19.1 L of the same material as the concentrated L-tryptophan fermentation broth used in Example 1A was supplied into a thin film drying apparatus, respectively. The internal pressure of the thin film drying apparatus was adjusted, and the operating conditions of the thin film drying apparatus except for the internal pressure were maintained the same as in Example 1A. Next, the recovered wet granules were placed in a fluidized-bed granulator/dryer and dried according to Examples and Comparative Examples.

Experimental results for each condition of Examples and Comparative Examples are as shown in Table 4 below.

**[Table 4]**

| Division | | Example 1A | Example 1I | Comparative Example 1F | Comparative Example 1G |
|---|---|---|---|---|---|
| Facility condition s | Pressure | 0.1 atm | 0.4 atm | 0.6 atm | 0.05 atm |
| | Steam temperat ure | 47°C | 77°C | 87°C | 34°C |
| Wet granules | Formatio n of granules | ○ | ○ | ○ | ○ |
| | Solid content | 92% | 89% | 90% | 94% |
| | Size | 400~700 µm | 400~700 µm | 400~700 µm | 300~500 µm |
| | Weight | 5.48 kg | 5.68 kg | 5.63 kg | 5.02 kg |
| Wet granules are placed in a laboratory fluidized-bed granulator/dryer and dried (discharged moisture 1%) | | | | | |
| Amino acid mixed solid | Weight | 5.04 kg | 5.05 kg | 5.06 kg | 4.72 kg |
| | Yield | 97.7% | 98.0% | 98.2% | 91.6% |

As a result of the experiment, it was confirmed that more dust was transported to the bag filter and the process yield decreased as the pressure in the thin film drying apparatus became lower but moisture drying was performed at a relatively high temperature and browning due to the high temperature occurred when the pressure in the thin film drying apparatus was high. Specifically, in a case where the pressure in the thin film drying apparatus exceeded about 0.05 atm (Comparative Example 1F), browning was confirmed in the amino acid mixed solid produced. In a case where the pressure in the thin film drying apparatus was less than about 0.6 atm (Comparative Example 1G), it was confirmed that dust was transported to the bag filter of the thin film drying apparatus and thus the process yield decreased to about 91.6%.

### Experimental Example 1-5. Preparation of granules using thin film drying apparatus and mixed-type granulator

Granules were formed using the L-tryptophan concentrate recovered in Example 1A by a thin film drying apparatus and a mixed-type granulator.

In Example 1J, 18.5 L of 19.1 L of the L-tryptophan concentrate recovered in Example 1A was placed in a thin film drying apparatus (JANGWOO MACHINERY CO., LTD., Horizontal type TFD), and drying was performed. The operating conditions of the thin film drying apparatus were the same as in Example 1A, but the introduction speed of the concentrate was lowered, and the discharged dried product weighed 5.03 kg, and had a purity of 67%, a solid content of 96%, and an average particle size of 200 µm to 300 µm. The discharged dried product was introduced into a mixed-type granulator (Lodige, Mix-Granulator) as a seed, and 0.6 L of the remained L-tryptophan concentrate was introduced into a mixed-type granulator using a metering feed pump (EYELA, RP-2100). Wet granules prepared in the mixed-type granulator weighed 5.68 kg, and had a purity of 67% and a solid content of 88%. The wet granules were placed in a laboratory fluidized-bed granulator/dryer and dried. The recovered wet granules weighed 4.98 kg, the recovery rate was 96.6%, the purity was 67% and the solid content was 99%.

Next, in Comparative Example 1H, 1.6 L of the L-tryptophan concentrate recovered in Example 1A and 9.5 kg of seeds (purity 65%, solid content 99%, average particle size 200 µm to 300 µm) were not introduced into a thin film drying apparatus but were directly introduced into a mixed-type granulator (Lodige, Mix-Granulator) to prepare wet granules. The L-tryptophan concentrate was introduced into the mixed-type granulator using a metering feed pump (EYELA, RP-2100). The wet granules weighed 11.15 kg, and had a purity of 65.1% and a solid content of 88%. The prepared wet granules were placed in a laboratory fluidized-bed granulator/dryer and dried. The recovered wet granules weighed 9.79 kg, the recovery rate was 98.8%, the purity was 65.1% and the solid content was 99%.

Through Example 1J, it was confirmed that a concentrate having a high water content could be prepared into fine granules using a thin film drying apparatus and the fine granules could be used as a seed in a mixed-type granulator.

### Example 2A. Preparation of amino acid mixed solid containing L-valine

In order to prepare an amino acid mixed solid containing L-valine by the method according to the present disclosure, the L-valine fermentation broth was first recovered from the fermenter.

The recovered L-valine fermentation broth had a composition of L-valine concentration of 80 g/L, purity of 80%, and solid content of 9.8%. The recovered L-valine fermentation broth was introduced into a concentration pipe for pilot use (forced circulation type, manufacturer MAHN MIN Machinery Co., Ltd., Korea) and concentrated. The fermentation broth used for concentration was 900 L in total, and concentrated 6 times in accordance with the capacity of the concentration pipe, 150 L/1 time. Concentration was performed under concentration conditions of a pressure of 0.1 atm and a steam pressure of 3 atm until the solid concentration reached 28%. Concentration was performed only until the solid concentration reached 28% since gelation might occur and fluidity might be lost when the solid concentration was 28% or more. The concentration of the recovered concentrate was 237.3 g/L, the purity was 80%, the solid concentration was 28%, and the volume was 303.4 L. Into a thin film drying apparatus, 20.2 L of the recovered concentrate was supplied.

Drying and formulation processes were performed using a thin film drying apparatus (double jacket, horizontal type, manufacturer JANGWOO MACHINERY CO., LTD., Korea) to prepare wet granules. The operating conditions of the thin film drying apparatus were set to be the same as in Example 1A.

From the thin film drying apparatus, 6.58 kg of wet granules were recovered, and the recovered wet granules had a purity of 80%, solid content of 90%, and an average particle size of about 400 to 700 µm. The wet granules were placed in a fluidized-bed granulator/dryer (manufacturer GR Engineering) and dried. The recovery rate of the amino acid mixed solid recovered from the fluidized-bed granulator/dryer was 97.7%, the amino acid mixed solid recovered weighed 5.92 kg, the purity was 80%, and the solid content was 99%.

### Experimental Example 2-1. Comparison of results of preparation of amino acid mixed solid depending on solid content in wet granules recovered from thin film drying apparatus

In order to compare results of preparation of the amino acid mixed solid depending on the solid content in wet granules recovered from the thin film drying apparatus, amino acid mixed solids were prepared while varying the degree of moisture drying.

In Examples and Comparative Examples used in Experimental Example 2-1, 20.2 L of the same material as the concentrated L-valine fermentation broth used in Example 2A was supplied into a thin film drying apparatus, respectively. The operating conditions of the thin film drying apparatus were maintained the same as in Example 2A, and the introduction speed was adjusted. Next, the recovered wet granules were placed in a fluidized-bed granulator/dryer and dried according to Examples and Comparative Examples.

Experimental results for each condition of Examples and Comparative Examples are as shown in Table 5 below.

**[Table 5]**

| Division | | Example 2A | Example 2B | Example 2C | Comparative Example 2A | Comparative Example 2B |
|---|---|---|---|---|---|---|
| Wet granules | Formation of granules | ○ | ○ | ○ | X | X |
| | Solid content | 90% | 85% | 80% | 75% | 95% |
| | Size | 400~700 µm | 400~700 µm | 500~800 µm | - | 200~300 µm |
| | Weight | 6.58 kg | 6.98 kg | 7.44 kg | - | 6.18 kg |
| Wet granules are placed in a laboratory fluidized-bed granulator/dryer and dried (discharged moisture 1%) | | | | | | |
| Amino acid mixed solid | Weight | 5.92 kg | 5.93 kg | 5.96 kg | - | 5.82 kg |
| | Yield | 97.7% | 97.9% | 98.3% | - | 96.0% |

As a result of the experiment, it was confirmed that granules are not formed when the solid content in wet granules recovered from the thin film drying apparatus was less than 80% (Comparative Example 2A). As the solid content in wet granules increased, the formation of granules became more difficult, and an additional formulation process was required. When the solid content in the wet granules was more than 94% (Comparative Example 2B), granules were not formed.

### Experimental Example 2-2. Comparison of results of preparation of amino acid mixed solid depending on stirring linear speed of thin film drying apparatus

In Examples and Comparative Examples used in Experimental Example 2-2, 20.2 L of the same material as the concentrated L-valine fermentation broth used in Example 2A was supplied into a thin film drying apparatus (JANGWOO MACHINERY CO., LTD., horizontal type TFD), respectively. The stirring speed (linear speed) in the thin film drying apparatus was adjusted, and the operating conditions of the thin film drying apparatus except for the stirring speed were maintained the same as in Example 2A. Next, the recovered wet granules were placed in a fluidized-bed granulator/dryer and dried according to Examples and Comparative Examples.

Experimental results for each condition of Examples and Comparative Examples are as shown in Table 6 below.

**[Table 6]**

| Division | | Example 2A | Example 2D | Example 2E | Example 2F | Comparative Example 2C | Comparative Example 2D |
|---|---|---|---|---|---|---|---|
| Linear speed | | 8 m/s | 5 m/s | 11 m/s | 14 m/s | 18 m/s | 3 m/s |
| Wet granules | Formatio n of granules | ○ | ○ | ○ | ○ | X | X |
| | Solid content | 90% | 87% | 92% | 94% | 96% | |
| | Size | 400~700 µm | 400~700 µm | 300~600 µm | 300~500 µm | 200~300 µm | |
| | Weight | 6.58 kg | 6.82 kg | 6.43 kg | 6.27 kg | 6.09 kg | |
| Wet granules are placed in a laboratory fluidized-bed granulator/dryer and dried (discharged moisture 1%) | | | | | | | |
| Amino acid mixed solid | Weight | 5.93 kg | 5.91 kg | 5.85 kg | 5.78 kg | | 5.93 kg |
| | Yield | 97.9% | 97.5% | 96.5% | 95.4% | | 97.9% |

As a result of the experiment, it was confirmed that as the stirring speed (linear speed) in the thin film drying apparatus increased, the amount of heat that the solids in the amino acid mixed solution can receive increased and the drying rate increased. However, when the linear speed exceeded 17 m/s, the amino acid mixed solution was overdried, the average particle size of wet granules decreased, and there was thus a problem in that the recovery rate decreased due to a significant loss of fine powder to the thin film drying apparatus and the bag filter of the fluidized-bed granulator/dryer. In contrast, at a linear speed of less than 4 m/s, the thin film formation rate decreased inside the thin film drying apparatus, and the load on the stirring unit of the thin film drying apparatus increased during drying and thus stirring was not performed.

### Experimental Example 2-3. Comparison of results of preparation of amino acid mixed solid depending on pressure condition in thin film drying apparatus

In Examples and Comparative Examples used in Experimental Example 2-3, 20.2 L of the same material as the concentrated L-valine fermentation broth used in Example 2A was supplied into a thin film drying apparatus, respectively. The internal pressure of the thin film drying apparatus was adjusted, and the operating conditions of the thin film drying apparatus except for the internal pressure were maintained the same as in Example 2A. Next, the recovered wet granules were placed in a fluidized-bed granulator/dryer and dried according to Examples and Comparative Examples.

Experimental results for each condition of Examples and Comparative Examples are as shown in Table 7 below.

**[Table 7]**

| Division | | Example 2A | Example 2G | Comparative Example 2E | Comparative Example 2F |
|---|---|---|---|---|---|
| Facility conditions | Pressure | 0.1 atm | 0.4 atm | 0.6 atm | 0.05 atm |
| | Steam temperat ure | 47°C | 77°C | 87°C | 34°C |
| Wet granules | Formatio n of granules | ○ | ○ | ○ | ○ |
| | Solid content | 90% | 88% | 86% | 97% |
| | Size | 400~700 µm | 400~700 µm | 400~700 µm | 200~300 µm |
| | Weight | 6.58 kg | 6.75 kg | 6.93 kg | 5.75 kg |
| Wet granules are placed in a laboratory fluidized-bed granulator/dryer and dried (discharged moisture 1%) | | | | | |
| Amino acid mixed solid | Weight | 5.92 kg | 5.95 kg | 5.96 kg | 5.48 kg |
| | Yield | 97.7% | 98.2% | 98.4% | 90.5% |

As a result of the experiment, it was confirmed that more dust was transported to the bag filter and the process yield decreased as the pressure in the thin film drying apparatus became lower but moisture drying was performed at a relatively high temperature and browning due to the high temperature occurred when the pressure in the thin film drying apparatus was high. Specifically, in a case where the pressure in the thin film drying apparatus exceeded about 0.05 atm (Comparative Example 2E), browning was confirmed in the amino acid mixed solid produced. In a case where the pressure in the thin film drying apparatus is less than about 0.6 atm (Comparative Example 2F), it was confirmed that dust passes to the bag filter of the thin film drying apparatus and thus the process yield decreased to about 90.5%.

### Experimental Example 2-4. Preparation of granules using thin film drying apparatus and mixed-type granulator

Granules were formed using the L-valine concentrate recovered in Example 2A by a thin film drying apparatus and a mixed-type granulator.

In Example 2H, 19.4 L of 20.2 L of the L-valine concentrate recovered in Example 2A was placed in a thin film drying apparatus (JANGWOO MACHINERY CO., LTD., Horizontal type TFD), and drying was performed. The operating conditions of the thin film drying apparatus were the same as in Example 2A, but the introduction speed of the concentrate was lowered, and the discharged dried product weighed 5.93 kg, and had a purity of 80%, a solid content of 95%, and an average particle size of 200 µm to 300 µm. The discharged dried product was introduced into a mixed-type granulator (Lodige, Mix-Granulator) as a seed, and 0.9 L of the remained L-valine concentrate was introduced into a mixed-type granulator using a metering feed pump (EYELA, RP-2100). Wet granules prepared in the mixed-type granulator weighed 6.85 kg, and had a purity of 80% and a solid content of 86%. The wet granules were placed in a laboratory fluidized-bed granulator/dryer and dried. The recovered wet granules weighed 5.86 kg, the recovery rate was 96.7%, the purity was 80% and the solid content was 99%.

Next, in Comparative Example 2G, 1.7 L of the L-valine concentrate recovered in Example 2A and 8.0 kg of seeds (purity 78%, solid content 99%, average particle size 200 µm to 300 µm) were not introduced into a thin film drying apparatus but were directly introduced into a mixed-type granulator (Lodige, Mix-Granulator) to prepare wet granules. The L-valine concentrate was introduced into the mixed-type granulator using a metering feed pump (EYELA, RP-2100). The wet granules weighed 9.75 kg, and had a purity of 78.1% and a solid content of 86%. The prepared wet granules were placed in a laboratory fluidized-bed granulator/dryer and dried. The recovered wet granules weighed 8.34 kg, the recovery rate was 98.4%, the purity was 78.1% and the solid content was 99%.

Through Example 2H, it was confirmed that a concentrate having a high water content can be prepared into fine granules using a thin film drying apparatus and the fine granules can be used as a seed in a mixed-type granulator.

### Example 3A. Preparation of amino acid mixed solid containing L-threonine

In order to prepare an amino acid mixed solid containing L-threonine by the method according to the present disclosure, an L-threonine fermentation broth was first recovered from the fermenter.

The recovered L-threonine fermentation broth had a composition of L-threonine concentration of 155 g/L, purity of 85%, and solid content of 17.7%. The recovered L-threonine fermentation broth was introduced into a concentration pipe for pilot use (forced circulation type, manufacturer MAHN MIN Machinery Co., Ltd., Korea) and concentrated. The fermentation broth used for concentration was 900 L in total, and concentrated 6 times in accordance with the capacity of the concentration pipe, 150 L/1 time. Concentration was performed under concentration conditions of a pressure of 0.1 atm and a steam pressure of 3 atm until the solid concentration reached 60%. Concentration was performed only until the solid concentration reached 60% since gelation might occur and fluidity might be lost when the solid concentration was 60% or more. The concentration of the recovered concentrate was 237.3 g/L, the purity was 85%, the solid concentration was 60%, and the volume was 246.5 L. Into a thin film drying apparatus, 16.4 L of the recovered concentrate was supplied.

Drying and formulation processes were performed using a thin film drying apparatus (double jacket, horizontal type, manufacturer JANGWOO MACHINERY CO., LTD., Korea) to prepare wet granules. The operating conditions of the thin film drying apparatus were set to be the same as in Example 1A.

From the thin film drying apparatus, 11.78 kg of wet granules were recovered, and the recovered wet granules had a purity of 85%, solid content of 92%, and an average particle size of about 300 to 600 µm. The wet granules were placed in a fluidized-bed granulator/dryer (manufacturer GR Engineering) and dried. The recovery rate of the amino acid mixed solid recovered from the fluidized-bed granulator/dryer was 97.9%, the recovered amino acid mixed solid weighed 10.82 kg, the purity was 85%, and the solid content was 99%.

### Experimental Example 3-1. Comparison of results of preparation of amino acid mixed solid depending on solid content in wet granules recovered from thin film drying apparatus

In order to compare results of preparation of the amino acid mixed solid depending on the solid content in wet granules recovered from the thin film drying apparatus, amino acid mixed solids were prepared while varying the degree of moisture drying.

In Examples and Comparative Examples used in Experimental Example 3-1, 16.4 L of the same material as the concentrated L-threonine fermentation broth used in Example 3A was supplied into a thin film drying apparatus, respectively. The operating conditions of the thin film drying apparatus were maintained the same as in Example 3A, and the introduction speed was adjusted. Next, the recovered wet granules were placed in a fluidized-bed granulator/dryer and dried according to Examples and Comparative Examples.

Experimental results for each condition of Examples and Comparative Examples are as shown in Table 8 below.

**[Table 8]**

| Division | | Example 3A | Example 3B | Example 3C | Comparative Example 3A | Comparative Example 3B |
|---|---|---|---|---|---|---|
| Wet granules | Formation of granules | ○ | ○ | ○ | X | X |
| | Solid content | 92% | 89% | 85% | 80% | 97% |
| | Size | 300~600 µm | 300~700 µm | 400~700 µm | - | 200~300 µm |
| | Weight | 11.76 kg | 12.13 kg | 12.76 kg | - | 10.88 kg |
| Wet granules are placed in a laboratory fluidized-bed granulator/dryer and dried (discharged moisture 1%) | | | | | | |
| Amino acid | Weight | 10.82 kg | 10.81 kg | 10.84 kg | - | 10.49 kg |
| mixed solid | Yield | 97.9% | 97.8% | 98.1% | - | 95.0% |

As a result of the experiment, it was confirmed that granules were not formed when the solid content in wet granules recovered from the thin film drying apparatus was less than 85% (Comparative Example 3A). As the solid content in wet granules increased, the formation of granules became more difficult, and an additional formulation process was required. When the solid content in the wet granules was more than 96% (Comparative Example 3B), granules were not formed.

### Experimental Example 3-2. Comparison of results of preparation of amino acid mixed solid depending on stirring linear speed of thin film drying apparatus

In Examples and Comparative Examples used in Experimental Example 3-2, 16.4 L of the same material as the concentrated L-threonine fermentation broth used in Example 3A was supplied into a thin film drying apparatus (JANGWOO MACHINERY CO., LTD., horizontal type TFD), respectively. The stirring speed (linear speed) in the thin film drying apparatus was adjusted, and the operating conditions of the thin film drying apparatus except for the stirring speed were maintained the same as in Example 3A. Next, the recovered wet granules were placed in a fluidized-bed granulator/dryer and dried according to Examples and Comparative Examples.

Experimental results for each condition of Examples and Comparative Examples are as shown in Table 9 below.

**[Table 9]**

| Division | | Example 3A | Example 3D | Example 3E | Example 3F | Comparative Example 3C | Comparative Example 3D |
|---|---|---|---|---|---|---|---|
| Linear speed | | 8 m/s | 5 m/s | 11 m/s | 14 m/s | 18 m/s | 3 m/s |
| Wet granules | Formatio n of granules | ○ | ○ | ○ | ○ | X | X |
| | Solid content | 92% | 90% | 94% | 96% | 97% | - |
| | Size | 300~600 µm | 400~700 µm | 300~600 µm | 300~400 µm | 200~300 µm | - |
| | Weight | 11.76 kg | 12.04 kg | 11.42 kg | 11.10 kg | 11.06 kg | - |
| Wet granules are placed in a laboratory fluidized-bed granulator/dryer and dried (discharged moisture 1%) | | | | | | | |
| Amino acid mixed solid | Weight | 10.82 kg | 10.81 kg | 10.67 kg | 10.59 kg | 10.55 kg | - |
| | Yield | 97.9% | 97.8% | 96.5% | 95.8% | 95.4% | - |

As a result of the experiment, it was confirmed that as the stirring speed (linear speed) in the thin film drying apparatus increased, the amount of heat that the solids in the amino acid mixed solution can receive increased and the drying rate increased. However, when the linear speed exceeded 17 m/s, the amino acid mixed solution was overdried, the average particle size of wet granules decreased, and there was thus a problem in that the recovery rate decreased due to a significant loss of fine powder to the thin film drying apparatus and the bag filter of the fluidized-bed granulator/dryer. In contrast, at a linear speed of less than 4 m/s, the thin film formation rate decreased inside the thin film drying apparatus, and the load on the stirring unit of the thin film drying apparatus increased during drying and thus stirring was not performed.

### Experimental Example 3-3. Comparison of results of preparation of amino acid mixed solid depending on pressure condition in thin film drying apparatus

In Examples and Comparative Examples used in Experimental Example 3-3, 16.4 L of the same material as the concentrated L-threonine fermentation broth used in Example 3A was supplied into a thin film drying apparatus, respectively. The internal pressure of the thin film drying apparatus was adjusted, and the operating conditions of the thin film drying apparatus except for the internal pressure were maintained the same as in Example 3A. Next, the recovered wet granules were placed in a fluidized-bed granulator/dryer and dried according to Examples and Comparative Examples.

Experimental results for each condition of Examples and Comparative Examples are as shown in Table 10 below.

**[Table 10]**

| Division | | Example 3A | Example 3G | Comparative Example 3E | Comparative Example 3F |
|---|---|---|---|---|---|
| Facility condition s | Pressure | 0.1 atm | 0.4 atm | 0.6 atm | 0.05 atm |
| | Steam temperat ure | 47°C | 77°C | 87°C | 34°C |
| Wet granules | Formatio n of granules | ○ | ○ | ○ | ○ |
| | Solid content | 92% | 90% | 89% | 97% |
| | Size | 300~600 µm | 400~700 µm | 400~700 µm | 200~300 µm |
| | Weight | 11.76 kg | 12.05 kg | 12.16 kg | 10.37 kg |
| Wet granules are placed in a laboratory fluidized-bed granulator/dryer and dried (discharged moisture 1%) | | | | | |
| Amino acid mixed solid | Weight | 10.82 kg | 10.86 kg | 10.82 kg | 9.94 kg |
| | Yield | 97.9% | 98.2% | 97.9% | 90.0% |

As a result of the experiment, it was confirmed that more dust passes to the bag filter and the process yield decreased as the pressure in the thin film drying apparatus became lower but moisture drying was performed at a relatively high temperature and browning due to the high temperature occurred when the pressure in the thin film drying apparatus was high. Specifically, in a case where the pressure in the thin film drying apparatus exceeded about 0.05 atm (Comparative Example 3E), browning was confirmed in the amino acid mixed solid produced. In a case where the pressure in the thin film drying apparatus is less than about 0.6 atm (Comparative Example 3F), it was confirmed that dust was transported to the bag filter of the thin film drying apparatus and thus the process yield decreased to about 90.0%.

### Experimental Example 3-4. Preparation of granules using thin film drying apparatus and mixed-type granulator

Granules were formed using the L-threonine concentrate recovered in Example 3A by a thin film drying apparatus and a mixed-type granulator.

In Example 3H, 15.8 L of 16.4 L of the L-threonine concentrate recovered in Example 3A was placed in a thin film drying apparatus (JANGWOO MACHINERY CO., LTD., Horizontal type TFD), and drying was performed. The operating conditions of the thin film drying apparatus were the same as in Example 3A, but the introduction speed of the concentrate was lowered, and the discharged dried product weighed 10.51 kg, and had a purity of 85%, a solid content of 96%, and an average particle size of 200 µm to 300 µm. The discharged dried product was introduced into a mixed-type granulator (Lodige, Mix-Granulator) as a seed, and 0.9 L of the remained L-threonine concentrate was introduced into a mixed-type granulator using a metering feed pump (EYELA, RP-2100). Wet granules prepared in the mixed-type granulator weighed 11.46 kg, and had a purity of 85% and a solid content of 93%. The wet granules were placed in a laboratory fluidized-bed granulator/dryer and dried. The recovered wet granules weighed 10.64 kg, the recovery rate was 96.3%, the purity was 85% and the solid content was 99%.

Next, in Comparative Example 3G, 1.4 L of the L-threonine concentrate recovered in Example 3A and 8.4 kg of seeds (purity 82.5%, solid content 99%, average particle size 200 µm to 300 µm) were not introduced into a thin film drying apparatus but were directly introduced into a mixed-type granulator (Lodige, Mix-Granulator) to prepare wet granules. The wet granules weighed 9.88 kg, and had a purity of 82.7% and a solid content of 93%. The L-threonine concentrate was introduced into the mixed-type granulator using a metering feed pump (EYELA, RP-2100). The prepared wet granules were placed in a laboratory fluidized-bed granulator/dryer and dried. The recovered wet granules weighed 9.15 kg, the recovery rate was 98.6%, the purity was 82.7% and the solid content was 99%.

Through Example 3H, it was confirmed that a concentrate having a high water content can be prepared into fine granules using a thin film drying apparatus and the fine granules can be used as a seed in a mixed-type granulator.

### Example 4A. Preparation of amino acid mixed solid containing L-isoleucine

In order to prepare an amino acid mixed solid containing L-isoleucine by the method according to the present disclosure, an L-isoleucine fermentation broth was first recovered from the fermenter.

The recovered L-isoleucine fermentation broth had a composition of L-isoleucine concentration of 36 g/L, purity of 58%, and solid content of 6.1%. The recovered L-isoleucine fermentation broth was introduced into a concentration pipe for pilot use (forced circulation type, manufacturer MAHN MIN Machinery Co., Ltd., Korea) and concentrated. The fermentation broth used for concentration was 900 L in total, and concentrated 6 times in accordance with the capacity of the concentration pipe, 150 L/1 time. Concentration was performed under concentration conditions of a pressure of 0.1 atm and a steam pressure of 3 atm until the solid concentration reached 31%. Concentration was performed only until the solid concentration reached 31% since gelation might occur and fluidity might be lost when the solid concentration was 31% or more. The concentration of the recovered concentrate was 180.2 g/L, the purity was 58%, the solid concentration was 31%, and the volume was 162.2 L. Into a thin film drying apparatus, 10.8 L of the recovered concentrate was supplied.

Drying and formulation processes were performed using a thin film drying apparatus (double jacket, horizontal type, manufacturer JANGWOO MACHINERY CO., LTD., Korea) to prepare wet granules. The operating conditions of the thin film drying apparatus were set to be the same as in Example 1A.

From the thin film drying apparatus, 4.19 kg of wet granules were recovered, and the recovered wet granules had a purity of 58%, solid content of 88%, and an average particle size of about 400 to 600 µm. The wet granules were placed in a fluidized-bed granulator/dryer (manufacturer GR Engineering) and dried. The recovery rate of the amino acid mixed solid recovered from the fluidized-bed granulator/dryer was 98.3%, the recovered amino acid mixed solid weighed 3.70 kg, the purity was 58%, and the solid content was 99%.

### Experimental Example 4-1. Comparison of results of preparation of amino acid mixed solid depending on solid content in wet granules recovered from thin film drying apparatus

In order to compare results of preparation of the amino acid mixed solid depending on the solid content in wet granules recovered from the thin film drying apparatus, amino acid mixed solids were prepared while varying the degree of moisture drying.

In Examples and Comparative Examples used in Experimental Example 4-1, 10.3 L of the same material as the concentrated L-isoleucine fermentation broth used in Example 34A was supplied into a thin film drying apparatus, respectively. The operating conditions of the thin film drying apparatus were maintained the same as in Example 4A, and the introduction speed was adjusted. Next, the recovered wet granules were placed in a fluidized-bed granulator/dryer and dried according to Examples and Comparative Examples.

Experimental results for each condition of Examples and Comparative Examples are as shown in Table 11 below.

**[Table 11]**

| Division | | Example 4A | Example 4B | Example 4C | Comparative Example 4A | Comparative Example 4B |
|---|---|---|---|---|---|---|
| Wet granules | Formation of granules | ○ | ○ | ○ | X | X |
| | Solid content | 88% | 85% | 82% | 79% | 96% |
| | Size | 400~600 µm | 400~700 µm | 400~700 µm | | 200~300 µm |
| | Weight | 4.19 kg | 4.33 kg | 4.50 kg | | 3.76 kg |
| Amino acid mixed solid | Weight | 3.70 kg | 3.68 kg | 3.69 kg | | 3.57 kg |
| | Yield | 98.3% | 97.8% | 98.1% | | 95.0% |

As a result of the experiment, it was confirmed that granules were not formed when the solid content in wet granules recovered from the thin film drying apparatus was less than 82% (Comparative Example 4A). As the solid content in wet granules increased, the formation of granules became more difficult, and an additional formulation process was required. When the solid content in the wet granules was more than 94% (Comparative Example 4B), granules were not formed.

### Experimental Example 4-2. Comparison results of preparation of of amino acid mixed solid depending on stirring linear speed of thin film drying apparatus

In Examples and Comparative Examples used in Experimental Example 4-2, 10.3 L of the same material as the concentrated L-isoleucine fermentation broth used in Example 4A was supplied into a thin film drying apparatus (JANGWOO MACHINERY CO., LTD., horizontal type TFD), respectively. The stirring speed (linear speed) in the thin film drying apparatus was adjusted, and the operating conditions of the thin film drying apparatus except for the stirring speed were maintained the same as in Example 4A. Next, the recovered wet granules were placed in a fluidized-bed granulator/dryer and dried according to Examples and Comparative Examples.

Experimental results for each condition of Examples and Comparative Examples are as shown in Table 12 below.

**[Table 12]**

| Division | | Example 4A | Example 4D | Example 4E | Example 4F | Comparative Example 4C | Comparative Example 4D |
|---|---|---|---|---|---|---|---|
| Linear speed | | 8 m/s | 5 m/s | 11 m/s | 14 m/s | 18 m/s | 3 m/s |
| Wet granules | Formatio n of granules | ○ | ○ | ○ | ○ | X | X |
| | Solid content | 88% | 86% | 91% | 94% | 96% | - |
| | Size | 400~600 µm | 400~700 µm | 300~500 µm | 300~400 µm | 200~300 µm | - |
| | Weight | 4.19 kg | 4.28 kg | 4.02 kg | 3.87 kg | 3.75 kg | |
| Wet granules are placed in a laboratory fluidized-bed granulator/dryer and dried (discharged moisture 1%) | | | | | | | |
| Amino acid mixed solid | Weight | 3.70 kg | 3.68 kg | 3.65 kg | 3.61 kg | 3.57 kg | - |
| | Yield | 98.3% | 97.9% | 97.1% | 96.0% | 94.8% | - |

As a result of the experiment, it was confirmed that as the stirring speed (linear speed) in the thin film drying apparatus increased, the amount of heat that the solids in the amino acid mixed solution can receive increased and the drying rate increased. However, when the linear speed exceeded 17 m/s, the amino acid mixed solution was overdried, the average particle size of wet granules decreased, and there was thus a problem in that the recovery rate decreased due to a significant loss of fine powder to the thin film drying apparatus and the bag filter of the fluidized-bed granulator/dryer. In contrast, at a linear speed of less than 4 m/s, the thin film formation rate decreased inside the thin film drying apparatus, and the load on the thin film drying apparatus stirring unit increased during drying and thus stirring was not performed.

### Experimental Example 4-3. Comparison of results of preparation of amino acid mixed solid depending on pressure condition in thin film drying apparatus

In Examples and Comparative Examples used in Experimental Example 4-3, 10.3 L of the same material as the concentrated L-isoleucine fermentation broth used in Example 4A was supplied into a thin film drying apparatus, respectively. The internal pressure of the thin film drying apparatus was adjusted, and the operating conditions of the thin film drying apparatus except for the internal pressure were maintained the same as in Example 4A. Next, the recovered wet granules were placed in a fluidized-bed granulator/dryer and dried according to Examples and Comparative Examples.

Experimental results for each condition of Examples and Comparative Examples are as shown in Table 13 below.

**[Table 13]**

| Division | | Example 4A | Example 4G | Comparative Example 4E | Comparative Example 4F |
|---|---|---|---|---|---|
| Facility condition s | Pressure | 0.1 atm | 0.4 atm | 0.6 atm | 0.05 atm |
| | Steam temperat ure | 47°C | 77°C | 87°C | 34°C |
| Wet granules | Formatio n of granules | ○ | ○ | ○ | ○ |
| | Solid content | 88% | 89% | 87% | 95.5% |
| | Size | 400~600 µm | 400~600 µm | 400~600 µm | 200~300 µm |
| | Weight | 4.19 kg | 4.14 kg | 4.23 kg | 3.62 kg |
| Wet granules are placed in a laboratory fluidized-bed granulator/dryer and dried (discharged moisture 1%) | | | | | |
| Amino acid mixed solid | Weight | 3.70 kg | 3.67 kg | 3.69 kg | 3.43 kg |
| | Yield | 98.3% | 97.6% | 98.0% | 91.1% |

As a result of the experiment, it was confirmed that more dust was transported to the bag filter and the process yield decreased as the pressure in the thin film drying apparatus became lower but moisture drying was performed at a relatively high temperature and browning due to the high temperature occurred when the pressure in the thin film drying apparatus was high. Specifically, in a case where the pressure in the thin film drying apparatus exceeded about 0.05 atm (Comparative Example 4E), browning was confirmed in the amino acid mixed solid produced. In a case where the pressure in the thin film drying apparatus is less than about 0.6 atm (Comparative Example 4F), it was confirmed that dust was transported to the bag filter of the thin film drying apparatus and thus the process yield decreased to about 91.1 %.

### Experimental Example 4-4. Preparation of granules using thin film drying apparatus and mixed-type granulator

Granules were formed using the L-isoleucine concentrate recovered in Example 4A by a thin film drying apparatus and a mixed-type granulator.

In Example 4H, 10.3 L of 10.8 L of the L-isoleucine concentrate recovered in Example 4A was placed in a thin film drying apparatus (JANGWOO MACHINERY CO., LTD., Horizontal type TFD), and drying was performed. The operating conditions of the thin film drying apparatus were the same as in Example 4A, but the introduction speed of the concentrate was lowered, and the discharged dried product weighed 3.64 kg, and had a purity of 58%, a solid content of 95.2%, and an average particle size of 200 µm to 300 µm. The discharged dried product was introduced into a mixed-type granulator (Lodige, Mix-Granulator) as a seed, and 0.5 L of the remained L-isoleucine concentrate was introduced into a mixed-type granulator using a metering feed pump (EYELA, RP-2100). Wet granules prepared in the mixed-type granulator weighed 4.17 kg, and had a purity of 58% and a solid content of 87%. The wet granules were placed in a laboratory fluidized-bed granulator/dryer and dried. The recovered wet granules weighed 3.63 kg, the recovery rate was 96.4%, the purity was 58% and the solid content was 99%.

Next, in Comparative Example 4G, 1.8 L of the L-isoleucine concentrate recovered in Example 4A and 13.1 kg of seeds (purity 56.5%, solid content 99%, average particle size 200 µm to 300 µm) were not introduced into a thin film drying apparatus but were directly introduced into a mixed-type granulator (Lodige, Mix-Granulator) to prepare wet granules. The wet granules weighed 15.13 kg, and had a purity of 56.6% and a solid content of 90%. The L-isoleucine concentrate was introduced into the mixed-type granulator using a metering feed pump (EYELA, RP-2100). The prepared wet granules were placed in a laboratory fluidized-bed granulator/dryer and dried. The recovered wet granules weighed 13.59 kg, the recovery rate was 98.8%, the purity was 56.6% and the solid content was 99%.

Through Example 4H, it was confirmed that a concentrate having a high water content can be prepared into fine granules using a thin film drying apparatus and the fine granules can be used as a seed in a mixed-type granulator.

### Example 5A. Preparation of amino acid mixed solid containing L-leucine

In order to prepare an amino acid mixed solid containing L-leucine by the method according to the present disclosure, an L-leucine fermentation broth was first recovered from the fermenter.

The recovered L-leucine fermentation broth had a composition of L-leucine concentration of 24 g/L, purity of 46%, and solid content of 5.6%. The recovered L-leucine fermentation broth was introduced into a concentration pipe for pilot use (forced circulation type, manufacturer MAHN MIN Machinery Co., Ltd., Korea) and concentrated. The fermentation broth used for concentration was 900 L in total, and concentrated 6 times in accordance with the capacity of the concentration pipe, 150 L/1 time. Concentration was performed under concentration conditions of a pressure of 0.1 atm and a steam pressure of 3 atm until the solid concentration reached 35%. Concentration was performed only until the solid concentration reached 35% since gelation might occur and fluidity might be lost when the solid concentration was 42% or more. The concentration of the recovered concentrate was 151.0 g/L, the purity was 44%, the solid concentration was 35%, and the volume was 142.2 L. Into a thin film drying apparatus, 10.5 L of the recovered concentrate was supplied.

Drying and formulation processes were performed using a thin film drying apparatus (double jacket, horizontal type, manufacturer JANGWOO MACHINERY CO., LTD., Korea) to prepare wet granules. The operating conditions of the thin film drying apparatus were set to be the same as in Example 1A.

From the thin film drying apparatus, 4.12 kg of wet granules were recovered, and the recovered wet granules had a purity of 43%, solid content of 87%, and an average particle size of about 400 to 600 µm. The wet granules were placed in a fluidized-bed granulator/dryer (manufacturer GR Engineering) and dried. The recovery rate of the amino acid mixed solid recovered from the fluidized-bed granulator/dryer was 97.2%, the recovered amino acid mixed solid weighed 3.60 kg, the purity was 43%, and the solid content was 99%.

### Experimental Example 5-1. Comparison of results of preparation of amino acid mixed solid depending on solid content in wet granules recovered from thin film drying apparatus

In order to compare results of preparation of the amino acid mixed solid depending on the solid content in wet granules recovered from the thin film drying apparatus, amino acid mixed solids were prepared while varying the degree of moisture drying.

In Examples and Comparative Examples used in Experimental Example 5-1, 10.0 L of the same material as the concentrated L-leucine fermentation broth used in Example 5A was supplied into a thin film drying apparatus, respectively. The operating conditions of the thin film drying apparatus were maintained the same as in Example 5A, and the introduction speed was adjusted. Next, the recovered wet granules were placed in a fluidized-bed granulator/dryer and dried according to Examples and Comparative Examples.

Experimental results for each condition of Examples and Comparative Examples are as shown in Table 14 below.

**[Table 14]**

| Division | | Example 5A | Example 5B | Example 5C | Comparative Example 5A | Comparative Example 5B |
|---|---|---|---|---|---|---|
| Wet granules | Formation of granules | ○ | ○ | ○ | X | X |
| | Solid content | 87% | 86% | 82% | 75% | 95% |
| | Size | 400~600 µm | 400~700 µm | 400~700 µm | | 200~300 µm |
| | Weight | 4.12 kg | 4.19 kg | 4.50 kg | | 3.69 kg |
| Wet granules are placed in a laboratory fluidized-bed granulator/dryer and dried (discharged moisture 1%) | | | | | | |
| Amino acid mixed solid | Weight | 3.60 kg | 3.63 kg | 3.69 kg | | 3.51 kg |
| | Yield | 97.2% | 96.4% | 98.1% | | 96.4% |

As a result of the experiment, it was confirmed that granules were not formed when the solid content in wet granules recovered from the thin film drying apparatus was less than 82% (Comparative Example 5A). As the solid content in wet granules increased, the formation of granules became more difficult, and an additional formulation process was required. When the solid content in the wet granules was more than 93% (Comparative Example 2B), granules were not formed.

### Experimental Example 5-2. Comparison of results of preparation of amino acid mixed solid depending on stirring linear speed of thin film drying apparatus

In Examples and Comparative Examples used in Experimental Example 5-2, 10.0 L of the same material as the concentrated L-leucine fermentation broth used in Example 5A was supplied into a thin film drying apparatus (JANGWOO MACHINERY CO., LTD., horizontal type TFD), respectively. The stirring speed (linear speed) in the thin film drying apparatus was adjusted, and the operating conditions of the thin film drying apparatus except for the stirring speed were maintained the same as in Example 5A. Next, the recovered wet granules were placed in a fluidized-bed granulator/dryer and dried according to Examples and Comparative Examples.

Experimental results for each condition of Examples and Comparative Examples are as shown in Table 15 below.

**[Table 15]**

| Division | | Example 5A | Example 5D | Example 5E | Example 5F | Comparative Example 5C | Comparative Example 5D |
|---|---|---|---|---|---|---|---|
| Linear speed | | 8 m/s | 5 m/s | 11 m/s | 14 m/s | 18 m/s | 3 m/s |
| Wet granules | Formatio n of granules | ○ | ○ | ○ | ○ | X | X |
| | Solid content | 87% | 83% | 88% | 93% | 96% | |
| | Size | 400~600 µm | 400~700 µm | 300~500 µm | 300~400 µm | 200~300 µm | |
| | Weight | 4.12 kg | 4.25 kg | 4.02 kg | 3.87 kg | 3.70 kg | |
| Wet granules are placed in a laboratory fluidized-bed granulator/dryer and dried (discharged moisture 1%) | | | | | | | |
| Amino acid mixed solid | Weight | 3.60 kg | 3.49 kg | 3.45 kg | 3.41 kg | 3.50 kg | - |
| | Yield | 97.2% | 97.9% | 97.1% | 96.0% | 94.0% | - |

As a result of the experiment, it was confirmed that as the stirring speed (linear speed) in the thin film drying apparatus increased, the amount of heat that the solids in the amino acid mixed solution can receive increased and the drying rate increased. However, when the linear speed exceeded 17 m/s, the amino acid mixed solution was overdried, the average particle size of wet granules decreased, and there was thus a problem in that the recovery rate decreased due to a significant loss of fine powder to the thin film drying apparatus and the bag filter of the fluidized-bed granulator/dryer. In contrast, at a linear speed of less than 4 m/s, the thin film formation rate decreased inside the thin film drying apparatus, and the load on the thin film drying apparatus stirring unit increased during drying and thus stirring was not performed.

### Experimental Example 5-3. Comparison of results of preparation of amino acid mixed solid depending on pressure condition in thin film drying apparatus

In Examples and Comparative Examples used in Experimental Example 5-3, 10.0 L of the same material as the concentrated L-leucine fermentation broth used in Example 5A was supplied into a thin film drying apparatus, respectively. The internal pressure of the thin film drying apparatus was adjusted, and the operating conditions of the thin film drying apparatus except for the internal pressure were maintained the same as in Example 5A. Next, the recovered wet granules were placed in a fluidized-bed granulator/dryer and dried according to Examples and Comparative Examples.

Experimental results for each condition of Examples and Comparative Examples are as shown in Table 16 below.

**[Table 16]**

| Division | | Example 5A | Example 5G | Comparative Example 5E | Comparative Example 5F |
|---|---|---|---|---|---|
| Facility condition s | Pressure | 0.1 atm | 8 m/s | 8 m/s | 8 m/s |
| | Steam temperat ure | 47°C | 0.4 atm | 0.6 atm | 0.05 atm |
| Wet granules | Formatio n of granules | ○ | 77°C | 87°C | 34°C |
| | Solid content | 87% | ○ | ○ | ○ |
| | Size | 400~600 µm | 88% | 87% | 94% |
| | Weight | 4.12 kg | 400~600 µm | 400~600 µm | 200~300 µm |
| Wet granules are placed in a laboratory fluidized-bed granulator/dryer and dried (discharged moisture 1%) | | | | | |
| Amino acid mixed solid | Weight | 3.60 kg | 3.46 kg | 3.53 kg | 3.28 kg |
| | Yield | 97.2% | 97.2% | 98.0% | 93.1% |

As a result of the experiment, it was confirmed that more dust passes to the bag filter and the process yield decreased as the pressure in the thin film drying apparatus became lower but moisture drying was performed at a relatively high temperature and browning due to the high temperature occurred when the pressure in the thin film drying apparatus was high. Specifically, in a case where the pressure in the thin film drying apparatus exceeded about 0.05 atm (Comparative Example 5E), browning was confirmed in the amino acid mixed solid produced. In a case where the pressure in the thin film drying apparatus was less than about 0.6 atm (Comparative Example 5F), it was confirmed that dust was transported to the bag filter of the thin film drying apparatus and thus the process yield decreased to about 93.1%.

### Experimental Example 5-4. Preparation of granules using thin film drying apparatus and mixed-type granulator

Granules were formed using the L-leucine concentrate recovered in Example 5A by a thin film drying apparatus and a mixed-type granulator.

In Example 5H, 10.0 L of 10.5 L of the L-leucine concentrate recovered in Example 5A was placed in a thin film drying apparatus (JANGWOO MACHINERY CO., LTD., Horizontal type TFD), and drying was performed. The operating conditions of the thin film drying apparatus were the same as in Example 5A, but the introduction speed of the concentrate was lowered, and the discharged dried product weighed 3.69 kg, and had a purity of 44%, a solid content of 94.5%, and an average particle size of 200 µm to 300 µm. The discharged dried product was introduced into a mixed-type granulator (Lodige, Mix-Granulator) as a seed, and 0.5 L of the remained L-leucine concentrate was introduced into a mixed-type granulator using a metering feed pump (EYELA, RP-2100). Wet granules prepared in the mixed-type granulator weighed 4.19 kg, and had a purity of 44% and a solid content of 86%. The wet granules were placed in a laboratory fluidized-bed granulator/dryer and dried. The recovered wet granules weighed 3.63 kg, the recovery rate was 96.4%, the purity was 43% and the solid content was 99%.

Next, in Comparative Example 5G, 2.0 L of the L-leucine concentrate recovered in Example 5A and 15.3 kg of seeds (purity 43%, solid content 99%, average particle size 200 µm to 300 µm) were not introduced into a thin film drying apparatus but were directly introduced into a mixed-type granulator (Lodige, Mix-Granulator) to prepare wet granules. The L-leucine concentrate was introduced into the mixed-type granulator using a metering feed pump (EYELA, RP-2100). The wet granules weighed 18.10 kg, and had a purity of 43% and a solid content of 88%. The prepared wet granules were placed in a laboratory fluidized-bed granulator/dryer and dried. The recovered wet granules weighed 16.05 kg, the recovery rate was 98.8%, the purity was 43% and the solid content was 99%.

Through Example 5H, it was confirmed that a concentrate having a high water content can be prepared into fine granules using a thin film drying apparatus and the fine granules can be used as a seed in a mixed-type granulator.

Based on the above description, it will be understood by those skilled in the art that the present disclosure may be implemented in a different specific form without changing the technical spirit or essential characteristics thereof. Therefore, it should be understood that the above embodiment is not limitative, but illustrative in all aspects. The scope of the disclosure is defined by the appended claims rather than by the description preceding them, and therefore all changes and modifications that fall within metes and bounds of the claims or equivalents of such metes and bounds are intended to be embraced by the claims.

## Claims

1. A method for preparing an amino acid mixed solid, the method comprising:
a first step of preparing an amino acid mixed solution containing an amino acid;
a second step of stirring the amino acid mixed solution to form a thin film, and drying and pulverizing the formed thin film to prepare wet granules; and
a third step of drying the wet granules to prepare an amino acid mixed solid in a granular formulation,
wherein, the second step is conducted for drying the amino acid mixed solution so that a solid concentration in the wet granules is within a granulation concentration range .

2. The method for preparing an amino acid mixed solid of claim 1,
further comprising a step of preparing granules using a mixed-type granulator after the second step has been performed and before the third step is performed,
wherein at least a portion of the wet granules prepared in the second step is used as a seed for preparing the granules in the mixed-type granulator.

3. The method for preparing an amino acid mixed solid of claim 1,
wherein the second step is conducted for controlling the granulation concentration range depending on the kind of amino acids.

4. The method for preparing an amino acid mixed solid of claim 1,
wherein the amino acid includes at least one selected from the group consisting of L-threonine, L-tryptophan, L-valine, L-isoleucine, and L-leucine.

5. The method for preparing an amino acid mixed solid of claim 1,
wherein the first step includes preparing the amino acid mixed solution by a fermentation method using at least one selected from a microorganism of the genus *Brevibacterium, Corynebacterium, Escherichia, Serratia, Erwinia, Enterobacteria, Streptomyces*, or *Pseudomonas* or an artificial mutant thereof.

6. The method for preparing an amino acid mixed solid of claim 1,
wherein a linear speed to stir the amino acid mixed solution in the second step is 4 m/s to 17 m/s.

7. The method for preparing an amino acid mixed solid of claim 1,
wherein the second step is performed at 0.05 atm to 0.6 atm.

8. The method for preparing an amino acid mixed solid of claim 1,
wherein the third step includes a drying process using a fluidized-bed granulator/dryer.

9. The method for preparing an amino acid mixed solid of claim 1,
further comprising a process of concentrating the amino acid mixed solution after the first step has been performed and before the second step is performed.

10. An apparatus for preparing an amino acid mixed solid, the apparatus comprising:
a fermenter for producing an amino acid mixed solution through a fermentation process;
a thin film drying apparatus for drying and pulverizing the amino acid mixed solution supplied from the fermenter to prepare wet granules; and
a granule drying apparatus for drying the wet granules to prepare an amino acid mixed solid in a granular formulation,
wherein the thin film drying apparatus includes:
a stirring unit of the thin film drying apparatus for stirring the amino acid mixed solution to form a thin film provided from the amino acid mixed solution on a thin film drying apparatus inner wall; and
a heating unit for heating the thin film drying apparatus inner wall to dry the thin film.

11. The apparatus for preparing an amino acid mixed solid of claim 10,
wherein the stirring unit of thin film drying apparatus and the thin film drying apparatus inner wall are isolated by a distance of 5 mm to 15 mm.

12. The apparatus for preparing an amino acid mixed solid of claim 10,
wherein the amino acid mixed solution is dried in the thin film drying apparatus so that a solid concentration in the wet granules is within a granulation concentration range, and
the granulation concentration range is controlled depending on the kind of amino acids.

13. The apparatus for preparing an amino acid mixed solid of claim 10,
further comprising a mixed-type granulator for preparing granules using at least a portion of the wet granules prepared in the thin film drying apparatus as a seed.
